# EUROPÄISCHE PATENTANMELDUNG

(11) **EP 0 726 080 A2**
(43) Veröffentlichungstag der Anmeldung: **14.08.1996**
(21) Anmeldenummer: 95119277.2
(22) Anmeldetag: 07.12.1995
(51) Int. Cl.: A61M 5/44

(54) **Vorrichtung zum Temperieren von Infusionsbeuteln**

(30) Priorität: 02.02.1995 DE 19503350
(71) Anmelder: Barkey, Volker, D-33619 Bielefeld (DE)
(72) Erfinder: Volker Barkey, D - 33619 Bielefeld (DE)
(74) Vertreter: TER MEER - MÜLLER - STEINMEISTER & PARTNER

(57) **Zusammenfassung**

Eine Vorrichtung zum Temperieren von Infusionsbeuteln während eines Infusionsvorganges umfaßt eine U-förmig gebogene Platte (10,40,50,60) und eine mit der Platte verbundene Heizeinrichtung (22,26,28,32) zur gesteuerten Erwärmung oder Warmhaltung eines in das U der Platte (10) eingelegten Infusionsbeutels (24).

## Beschreibung

Die Erfindung betrifft eine Vorrichtung zum Temperieren von Infusionsbeuteln während eines Infusionsvorganges.

Infusionslösungen werden zumeist in Flaschen oder Beutein an einem Gestell in einer Position oberhalb eines Patienten aufgehängt, so daß die Lösung über Schläuche durch Schwerkraft in den Körper des Patienten gelangen kann. Dies führt in vielen Fällen, etwa bei längeren Operationen, bei älteren Patienten oder auch bei länger andauernder Verabreichung, bei der sich Infusionslösungen mehr oder weniger auf Raumtemperatur befinden, zu einer erheblichen Unterkühlung der Patienten, die vor allem von körperlich geschwächten Patienten nur langsam wieder ausgeglichen werden kann.

Es sind daher verschiedene Einrichtungen zum Warmhalten oder Erwärmen von Infusionslösungen bekannt. Beispiele aus dem Stand der Technik veranschaulichen das deutsche Gebrauchsmuster G 92 01 792 und die GB-A 2 248 106. Die bekannten Vorrichtungen erfüllen die gestellten Anforderungen zumeist nur unzureichend.

Oft sind der Aufbau und die Handhabung kompliziert und wenig praxisgerecht. Auch die Sicherung der Sterilität bereitet vielfach Schwierigkeiten. Sofern die bekannten Einrichtungen ganz oder teilweise als Einwegartikel ausgebildet sind, widersprechen sie dem zunehmend an Bedeutung gewinnenden Aspekt der Müllvermeidung.

Der Erfindung liegt die Aufgabe zugrunde, eine gattungsgemäße Vorrichtung im Aufbau und in der Handhabung besonders einfach, in der Herstellung kostengünstig und hinsichtlich der Reinigung und Sterilhaltung unproblematisch auszubilden.

Diese Aufgabe wird erfindungsgemäß bei einer Vorrichtung der obigen Art dadurch gelöst, daß die Vorrichtung eine U-förmig gebogene Platte und eine mit der Platte verbundene Heizeinrichtung zur gesteuerten Erwärmung oder Warmhaltung eines in das U in der Platte eingelegten Infusionsbeutels umfaßt.

Ein in die Vorrichtung eingelegter Infusionsbeutel steht mehr oder weniger mit den beiden Armen oder Schenkeln des U in Berührung, so daß eine Temperierung der Infusionslösung durch Wärmeleitung erfolgen kann. Je nach Art der verwendeten Heizeinrichtung wird auch eine gewisse Übertragung durch Wärmestrahlung und Konvektion stattfinden.

Die Heizeinrichtung kann Heizdrähte oder eine Heizfolie umfassen, die innen oder außen an der Platte angebracht sind. Eine gute Wärmeverteilung ergibt sich, wenn die Platte aus Metall oder einem entsprechend wärmeleitenden Material besteht und die Heizeinrichtung auf der Außenseite der Platte in der Form einer Heizfolie angebracht, zum Beispiel verklebt ist.

Die meisten Infusionsbeutel haben eine im wesentlichen rechteckige Form, während die Position der Auslässe unterschiedlich ist. Befindet sich jeweils ein Auslaß oder Einlaß in den beiden unteren Ecken, so ist es zweckmäßig die Platte so auszubilden, daß das U der Platte am Boden des U von beiden Seiten her leicht ausgenommen ist. Soll mit Beutein gearbeitet werden, die Auslässe in der Mitte ihres Bodens aufweisen, so ist im Boden der U-förmigen Platte eine Öffnung oder auch eine von einer Seite her bis hin zur Mitte eintretende Ausnehmung vorzusehen.

Die Schenkel des U können unterschiedlich lang und unterschiedlich breit sein. Zweckmäßigerweise ist an einem der Schenkel eine Aufhängevorrichtung vorgesehen. Dieser Schenkel kann an seinem oberen Ende bis zur senkrechten Mittelebene des U umgebogen sein, so daß die Aufhängung symmetrisch in bezug auf die Vorrichtung erfolgt und die Vorrichtung im Gebrauch senkrecht hängt.

Eine zusätzliche Erwärmung und zugleich Lagesicherung des Beutels, die beispielsweise bei Verwendung in fahrenden Krankenfahrzeugen eine Rolle spielen kann, läßt sich erreichen durch umlegbare Laschen oder Klappen, die das U von beiden Seiten her nach dem Einlegen eines Beutels schließen.

Im folgenden werden bevorzugte Ausführungsbeispiele der Erfindung anhand der beigefügten Zeichnung näher erläutert.
- Fig. 1: ist eine perspektivische Darstellungsform einer erfindungsgemäßen Vorrichtung mit einem eingelegten Infusionsbeutel;
- Fig. 2: ist eine schematische Seitenansicht einer zweiten Ausführungsform der Vorrichtung;
- Fig. 3 und 4: zeigen weitere Ausführungsformen der Erfindung in perspektivischer Darstellung.

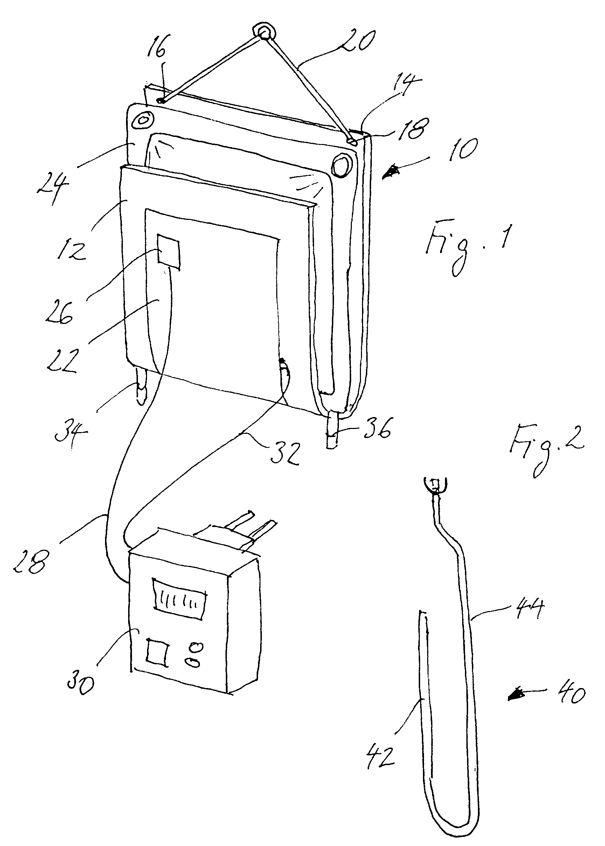

Eine erfindungsgemäße Vorrichtung gemäß Fig. 1 weist als Grundbestandteil eine U-förmig gebogene Platte 10 mit zwei parallelen, aufragenden Armen oder Schenkeln 12,14 auf. Bei dem dargestellten Beispiel ist der dem Betrachter zugewandte, vordere Schenkel geringfügig kürzer und unter Umständen auch schmaler als der hintere Schenkel 14. Der hintere Schenkel 14 weist an den beiden oberen Ecken Befestigungsösen 16,18 zur Befestigung eines Bügels 20 auf, der zum Aufhängen der Vorrichtung an einem nicht gezeigten Gestell in einer Position oberhalb eines nicht gezeigten Patienten auf. Die Platte 10 besteht vorzugsweise aus einem Metall oder einem ähnlich gut wärmeleitenden Material. Auf der Außenseite ist bei der gezeigten Ausführungsform eine Heizfolie 22 auf die beiden Schenkel 12,14 der Platte 10 aufgeklebt. Anstelle der Heizfolie können auch Heizdrähte vorgesehen sein. An der Platte können auch Kanäle ausgebildet sein, die von einem Heizmedium durchströmt werden. Die Art der Temperierung der Platte ist beliebig. Durch Anbringen einer Heizfolie auf der Außenseite der Platte 10 ergibt sich eine gute Wärmeverteilung, da die Platte 10 in ihrer Gesamtheit gleichmäßig erwärmt wird. Ein Infusionsbeutel 24 ist von oben in die U-förmige Platte 10 eingelegt. Der Abstand der Schenkel 12,14 der Platte 10 ist so gewählt daß der Infusionsbeutel 24 beide Schenkel möglichst großflächig berührt.

Auf der dem Betrachter in Fig. 1 zugewandten Seite des Schenkels 12 ist im Bereich der Heizfolie 22 ein Temperatursensor 26 befestigt.

Der Temperatursensor 26 ist über eine Leitung mit einem Netz- und Steuergerät 30 verbunden, das seinerseits die Heizfolie 22 über eine weitere Leitung 32 mit Heizstrom versorgt.

Die erfindungsgemäße Vorrichtung ist äußerst einfach aufgebaut, kostengünstig herzustellen und leicht zu reinigen. Das Einlegen eines Infusionsbeutels 24 bereitet keine Schwierigkeiten.

Bei dem in Fig. 1 gezeigten Beispiel wird davon ausgegangen, daß der Infusionsbeutel 24 geringfügig breiter ist als die Platte 10, so daß zwei Auslässe 34,36 des Infusionsbeutels beidseitig der Platte 10 nach unten ragen.

Alternative Ausführungsformen sollen insoweit später erläutert werden.

Fig. 2 zeigt in einer schematischen Seitenansicht eine weitere Vorrichtung gemäß der Erfindung mit einer U-förmig gebogenen Platte 40 mit zwei parallelen, senkrecht aufragenden Schenkeln 42,44. In Abweichung von Fig. 1 ist die Platte 44 an ihrem oberen Ende verlängert und etwa bis hin zur nicht dargestellten Mittelebene des U abgekröpft, so daß sich die Befestigungsösen 46 oder sonstige Aufhängungsorgane senkrecht über dem Schwerpunkt der Anordnung aus Beutel und Platte befinden und die gesamte Vorrichtung mit dem Beutel gerade herabhängt.

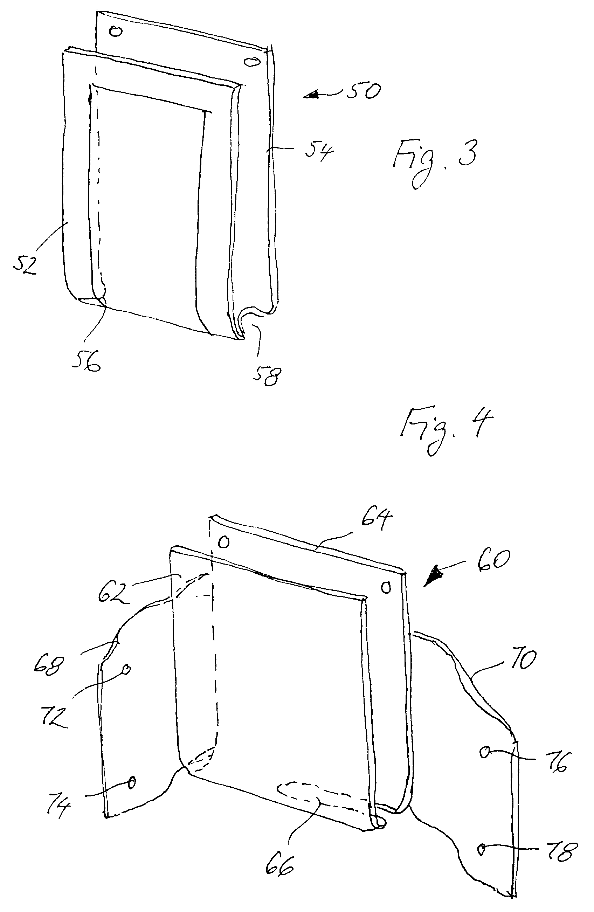

Fig. 3 zeigt eine weitere Ausführungsform mit einer gebogenen Platte 50 mit einem vorderen und einem hinteren Schenkel 52,54 und entspricht insoweit den zuvor geschilderten Ausführungsformen. In Abweichung von Fig. 1 weist die Platte am Boden des U auf beiden Seiten ausgerundete Ausnehmungen 56,58 auf. Diese Ausführungsform eignet sich für die Aufnahme von Infusionsbeuteln, deren Auslässe enger beieinanderliegen als diejenigen des Beutels gemäß Fig. 1.

Bei der in Fig. 4 gezeigten Ausführungsform ist die Aufnahme von Infusionsbeuteln vorgesehen, deren Auslässe sich im wesentlichen in der Mitte ihres unteren Randes befinden. Eine in Fig. 4 gezeigte Platte ist mit 60 und die beiden Schenkel sind mit 62,64 bezeichnet. Im Boden des U tritt von rechts in Fig. 1 eine längliche Ausnehmung 66 bis zur Mitte der Platte ein, so daß ein nicht gezeigter Infusionsbeutel mit in der Mitte abgehenden Auslässen in die Platte eingelegt oder von rechts in Fig. 4 eingeschoben werden kann.

Fig. 4 zeigt im übrigen eine weitere mögliche Ergänzung in der Form von zwei Laschen 68,70, die von dem hinteren Schenkel 64 nach beiden Seiten ausgehen und auf der vorderen Platte 62 aufeinandergelegt und mit Hilfe von schematisch angedeuteten Druckknöpfen 72,74,76,78 miteinander verbunden werden können. Anstelle der Druckknöpfe können Haken und Ösen, Klettbänder etc. verwendet werden. Durch die beiden Laschen 68,70 werden die beiden offenen Seiten der U-förmigen Platte 60 geschlossen, so daß im Inneren der Platte eine Art von Wärmekammer gebildet wird, die die Erwärmung von Infusionsbeuteln erleichtert.

Die Platte 10,40,50,60 muß nicht eine zusammenhängende, einstückige, geschlossene Platte sein. Sie kann Durchbrechungen aufweisen, aus mehreren Teilen zusammengesetzt sein und mehr oder weniger von der genauen U-Form im senkrechten Schnitt abweichen. Wichtig ist lediglich, daß eine Aufnahme für die Infusionsbeutel gebildet wird, in die diese von oben eingelegt werden können.

## Patentansprüche

1. Vorrichtung zum Temperieren von Infusionsbeuteln während eines Infusionsvorganges, dadurch **gekennzeichnet**, daß die Vorrichtung eine U-förmig gebogene Platte (10,40,50,60) und eine mit der Platte verbundene Heizeinrichtung (22,26,28,32) zur gesteuerten Erwärmung oder Warmhaltung eines in das U der Platte (10) eingelegten Infusionsbeutels (24) umfaßt.

2. Vorrichtung nach Anspruch 2, dadurch **gekennzeichnet**, daß die Vorrichtung Heizdrähte umfaßt.

3. Vorrichtung nach Anspruch 1, dadurch **gekennzeichnet**, daß die Heizeinrichtung eine Heizfolie (22) umfaßt.

4. Vorrichtung nach Anspruch 2 oder 3, dadurch **gekennzeichnet**, daß die Platte (10,40,50,60) wärmeleitend ist, und daß die Heizdrähte oder die Heizfolie (22) außen auf der Platte angebracht sind.

5. Vorrichtung nach Anspruch 4, dadurch **gekennzeichnet**, daß die Platte (10,40,50,60) aus Metall besteht.

6. Vorrichtung nach einem der vorhergehenden Ansprüche, dadurch **gekennzeichnet**, daß einer der Schenkel (12,14; 42,44; 52,54; 62,64) des U kürzer und/oder schmaler als der andere Schenkel ausgebildet ist.

7. Vorrichtung nach einem der vorhergehenden Ansprüche, dadurch **gekennzeichnet**, daß am Boden des U wenigstens auf einer Seite eine in die Platte eintretende Ausnehmung (56,58,66) vorgesehen ist.

8. Vorrichtung nach einem der vorhergehenden Ansprüche, dadurch **gekennzeichnet**, daß einer der Schenkel (12,14; 42,44; 52,54; 62,64) der Platte (10,40,50,60) nach oben verlängert und bis hin zur senkrechten Mittelebene des U abgekröpft ist.

9. Vorrichtung nach einem der vorhergehenden Ansprüche, **gekennzeichnet** durch Laschen (68,70) oder Klappen zum Verschließen des U auf den gegenüberliegenden offenen Seiten.
